# EUROPEAN PATENT APPLICATION

(11) **EP 4 647 053 A1**
(43) Date of publication of application: **12.11.2025**
(21) Application number: 25175213.5
(22) Date of filing: 08.05.2025
(51) Int. Cl.: A61F 13/14, A41C 3/00, A61F 5/03

(54) **TRUNCAL COMPRESSION GARMENT FOR DIRECTING LYMPHATIC FLUID TO LYMPH VESSELS**

(30) Priority: 09.05.2024 US 202463644583 P; 03.06.2024 US 202463655251 P; 03.04.2025 US 202519169230
(71) Applicant: American Breast Care, LP, Marietta, GA 30067 (US)
(72) Inventor: Burt, Coco, Atlanta, 30345 (US)
(74) Representative: Wittmann, Günther

(57) **Abstract**

In a truncal compression garment (100) for use by a user, a body portion (120) has dimensions so as to be able to be wrapped at least partially around a length of the user's torso. A supporting band (130) is made of an elastic material that holds the body portion (120) in place while being worn by the user. An integrated structure (122) is disposed on an inside part of the body portion and is configured to promote movement and transport of lymph in the user towards available and undamaged lymph vessels. In a method of relieving lymphedema in a user having a lymphatic system that transports lymphatic fluid, an area of the user is massaged with a truncal compression garment (100) so as to move the lymphatic fluid. Protrusions (124) are used in the truncal compression garment to direct transport of lymph in the user towards available and undamaged lymph vessels.

## Description

### CROSS-REFERENCE TO RELATED APPLICATION(S)

This application claims the benefit of US Provisional Patent Application Serial Nos. 63/644,583, filed 05/09/2024, and 63/655,251, filed 06/03/2024, the entirety of which is hereby incorporated herein by reference.

### BACKGROUND OF THE INVENTION

### 1. Field of the Invention

The present invention relates to truncal compression garments and, more specifically, to a truncal compression garment for lymph drainage of the breast and chest area, axillary/lateral trunk area, shoulder area, and back.

### 2. Description of the Related Art

With the rise of certain breast cancer-related procedures that cause disruption to the lymphatic system, including but not limited to reconstructions, axillary lymph node dissections, and sentinel lymph node biopsies, the incidence of women developing truncal lymphedema is also increasing. Part of the treatment plan for truncal lymphedema involves wearing a compression vest or bra, in conjunction with thicker, channeled pressure accessories targeting the breast, axilla, and/or back to encourage certain lymph transport redirection. Traditionally, commercially available compression bras and thicker, channeled pressure accessories targeting these truncal areas are made separately and are not combined into a single truncal device. Although some existing garments contain textures and patterns, these textures insufficiently promote lymph drainage.

At nighttime, encouraging lymphatic fluid flow can be of particular concern. Currently, there are no products specifically targeting lymphatic fluid transport during nighttime in a bra-type garment. Nighttime compression treatment tends to have milder compression levels than daytime compression garments, yet such compression treatment also usually requires thick, channeled pads mentioned. There is a constant challenge for durable medical equipment suppliers to provide their customers with the appropriate items (a truncal compression garment and an additional channeled compression item) required for treatment.

Channeled pressure accessories used to encourage lymphatic fluid drainage in the trunk tend to have specific patterns and shapes that depend on where an individual's lymphedema presents itself and which of the individual's lymph nodes are damaged. However, these pads must be placed inside a garment or bra - either in a designated bra pocket or against the skin inside of the bra or vest. Despite the compression of the bra holding the pad against the body, the pad can still bunch up or shift so as not to encourage lymph drainage in the target area. Ensuring that the separate pad is in the right place every time it is worn can prove to be difficult. Also, for those with limited mobility due to surgeries or radiation that often accompany breast cancer treatment, placing the pad in the bra can require additional assistance. The channeled pressure pads used to treat truncal lymphedema are sometimes worn in garment pockets that wrap around the chest, underarm, or back. While the pressure pads are still compressed against the body with the support of the garment, there is a layer of fabric between the channel and the body thus minimizing the full potential of the channels. Finally, maintaining two separate items can result in a lost or misplaced unit.

Therefore, there is a need for a truncal compression garment including a bra with built-in pressure structures.

### SUMMARY OF THE INVENTION

The disadvantages of the prior art are overcome by the present invention which, in one aspect, is a truncal compression garment for use by a user having a torso and lymphatic fluid pathways. A body portion has dimensions so as to be able to be wrapped at least partially around a length of the user's torso. A supporting band is made of an elastic material that holds the body portion in place while being worn by the user. An integrated structure is disposed on an inside part of the body portion and is configured to promote movement and transport of lymph in the user towards available and undamaged lymph vessels.

In another aspect, the invention is a truncal compression garment having a front for use by a user having a torso and lymphatic fluid pathways that includes a body portion that has dimensions so as to be able to be wrapped at least partially around a length of the user's torso. A supporting band is made of an elastic material that holds the body portion in place while being worn by the user. An integrated structure is disposed on an inside part of the body portion and is configured to promote movement and transport of lymph in the user towards available and undamaged lymph vessels. A closure mechanism is configured to secure the body portion to the user. The closure is disposed on a front portion of the truncal compression garment.

In yet another aspect, the invention is a method of relieving lymphedema in a user having a lymphatic system that transports lymphatic fluid, in which an area of the user is massaged with a truncal compression garment so as to move the lymphatic fluid. Protrusions are used in the truncal compression garment to direct transport of lymph in the user towards available and undamaged lymph vessels.

These and other aspects of the invention will become apparent from the following description of the preferred embodiments taken in conjunction with the following drawings. As would be obvious to one skilled in the art, many variations and modifications of the invention may be effected without departing from the spirit and scope of the novel concepts of the disclosure.

### BRIEF DESCRIPTION OF THE FIGURES OF THE DRAWINGS

**FIGS. 1A-1B** are schematic drawings of one embodiment of a truncal compression garment.
FIG. 2 is a schematic drawing of a second embodiment of a truncal compression garment.
FIG. 3 is a schematic drawing of a third embodiment of a truncal compression garment.

### DETAILED DESCRIPTION OF THE INVENTION

A preferred embodiment of the invention is now described in detail. Referring to the drawings, like numbers indicate like parts throughout the views. Unless otherwise specifically indicated in the disclosure that follows, the drawings are not necessarily drawn to scale. The present disclosure should in no way be limited to the exemplary implementations and techniques illustrated in the drawings and described below. As used in the description herein and throughout the claims, the following terms take the meanings explicitly associated herein, unless the context clearly dictates otherwise: the meaning of "a," "an," and "the" includes plural reference, the meaning of "in" includes "in" and "on."

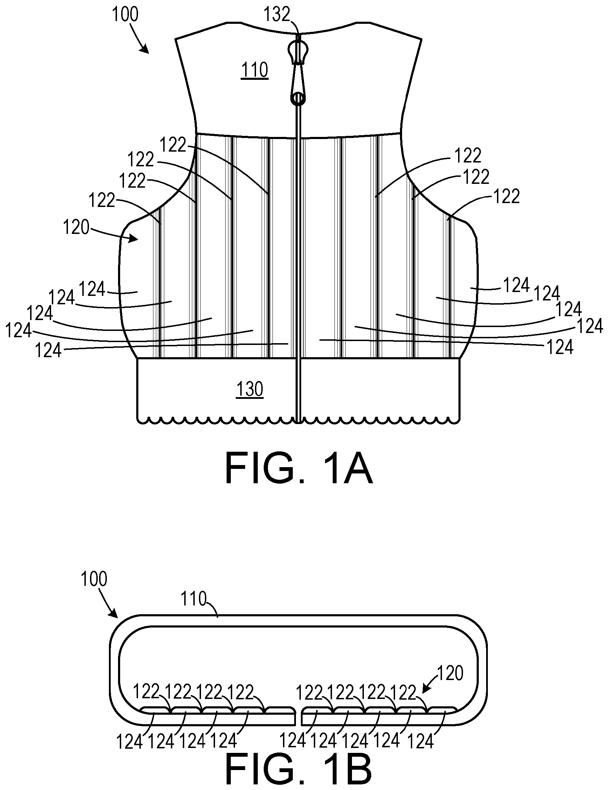

As shown in FIGS. 1A and 1B, one embodiment of a truncal compression garment **100** is adapted for use by a user having a torso. A body portion **110** has dimensions so as to be able to be wrapped at least partially around a length of the user's torso. A supporting band 130 is made of an elastic material and can be located under the channeling and around the torso so as to hold the garment in place. A front closure **132** secures the body portion to the user. An integrated structured portion **120** is disposed on an inside part of the body portion **110** and encourages direct flow of lymphatic fluid within the user's torso. The integrated structured portion **120** includes a plurality of protrusions **124** that define a corresponding plurality of channels **122**. The protrusions **124** massage areas on the user where normal flow of lymphatic fluid is restricted and the channels **122** allow for flow of the lymphatic fluid that has been re-routed by the massaging action.

In certain embodiments, the integrated structure **120** includes structures such as: channeling, protrusions, chip bags, ridges, or ribbing specifically designed patterns known to benefit lymphedema. The integrated structure **120** can include materials such as: foam, quilted fabric, silicone, gel, air, gel-infused foam, polymer beads, foam rubber, fiberfill or combinations thereof. The front closure can include a mechanical connection such as: a hook and loop fastener, a zipper, a plurality of snap buttons, a plurality of hook-sand-eyes, a plurality of magnetic closures, a drawstring, a buckle, or a strap, and combinations thereof. The integrated structure **120** can include an adjustable strap made of a material such as: slide adjusters, hook and eye adjusters, hook and loop (such as Velcro) adjusters, tie adjusters, elastic stretch adjusters, or ladder adjusters.

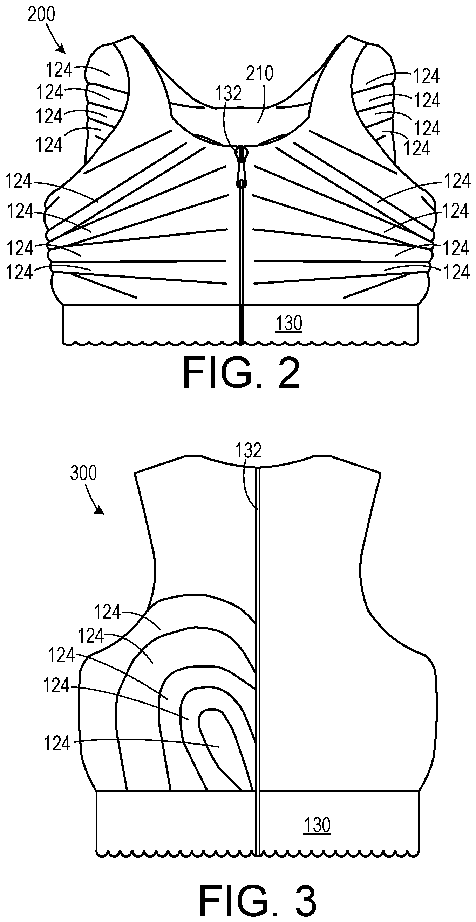

As shown in FIG. 2, one embodiment of a compression garment **200** can include sloped straight protrusions **124** and channels **122** that are used to direct lymphatic fluid to available and undamaged lymph vessels on the user's sides. Also, protrusions **124** and channels **122** can be included on a back portion **210** of the garment **200**.

The integrated structure portion **120** can cover both a left side of the user's torso and right side of the user's torso or, as shown in FIG. 3, only one side of the body portion of the garment **300**. When the integrated structure portion **120** covers only one side the body portion the front closure can be reversible in one embodiment. An elastic bottom portion **130** for holding the garment in place can also be affixed to the bottom of the integrated structure portion **120**.

In one embodiment, a truncal garment encircles the body and combines the benefits of the secure fit of a bra (at least one strap and supportive band) with the distinct structures for lymph drainage recommended for certain lymphedema drainage pathways in the truncal region for improved convenience and comfort while wearing. Applicant has discovered that combining the two above-described truncal garment technologies results in a truncal garment made with structures for durable and effective wearing.

In one representative embodiment, the garment has a shoulder strap adjustment to allow for a more appropriate fit to each wearer's torso. The front of the garment has a closure so the wearer can more easily wrap and secure the torso with the compression garment. In a typical embodiment, the garment does not provide levels of compression higher than 20mmHg according to a garment compression test done in accordance to RAL-GZ 387/1:2008-01, so it has relatively mild compression compatible with lounging or nighttime use. This garment can eliminate the need to have both a truncal compression garment and a channeled pad.

In another representative embodiment, the garment has a shoulder strap adjustment to allow for a more appropriate fit to each wearer's torso. Additionally, it can have channeled padding on only one side of the garment. The front of the garment has a reversible closure so the garment can be flipped depending on which side of the body requires the channeled attention. In a unilateral partial mastectomy for example, this configuration would allow less padding on the side of the body that was not affected, thereby minimizing discomfort that can accompany layers of foam or fabric. This reversible closure ensures minimal inventory for retailers stocking and fitting these devices for various wearers. The reversible front closure allows the wearer to wrap and secure the torso with the compression garment more easily.

The garment can employ structures that are designed to encourage lymph drainage from the breast, axilla, and back, which can include the following:
- Foam: Foam padding, often made from materials like polyurethane foam, memory foam, or latex foam, is widely used in medically padded devices due to its ability to provide cushioning and support while conforming to the body's contours.
- Gel: Gel padding is another common option, particularly in devices intended to reduce pressure and prevent pressure sores. Gel padding distributes pressure evenly and can help dissipate heat.
- Air: Air-filled cushions or padding are used to provide adjustable support and pressure relief. These can be adjusted to accommodate individual comfort and pressure distribution needs.
- Silicone: Silicone padding is often used in medical braces and orthopedic devices due to its flexibility, durability, and hypoallergenic properties. Silicone padding can provide cushioning while allowing for a certain degree of compression and movement.
- Gel-infused foam: Combining the benefits of foam and gel, gel-infused foam padding offers both support and pressure relief. This type of padding is often used in mattresses, wheelchair cushions, and orthopedic braces.
- Fiberfill: Fiberfill padding, typically made from polyester fibers, is soft and lightweight, making it suitable for padding in medical braces, splints, and garments. It provides cushioning while allowing for breathability.
- Polymer beads: These small beads, often made from polystyrene or similar materials, can be used as fillings in cushions or pads to provide adjustable support and comfort.
- Foam rubber: Foam rubber, also known as latex foam or sponge rubber, is another option for padding in medically padded devices. It offers cushioning and support and is resistant to mildew and bacteria.

Front closures for the garment can include the following:
- Hook-and-Loop (e.g., Velcro^{®}: This closure system uses hook-and-loop fasteners, to secure the garment. It allows for adjustable fit and easy adjustment, making it suitable for individuals with varying body shapes and sizes. Hook-and-loop closures are often found on compression wraps, braces, and post-surgical garments.
- Zipper: Zippers provide a secure closure and are relatively easy to operate. They are commonly used on compression garments, post-surgical bras, and orthopedic braces. Some medical garments feature zipper closures with protective fabric flaps or backing to prevent skin irritation or chafing.
- Snap Buttons: Snap buttons offer a secure closure and can be easier to manipulate than zippers for some individuals. They are commonly used on compression vests, abdominal binders, and post-surgical garments. Snap buttons provide adjustable fit options and can withstand repeated use.
- Hook-and-Eye: Similar to the closures found on bras, hook-and-eye closures feature hooks on one side of the garment and fabric loops (eyes) on the other side. They provide a secure closure and are often used on compression bras, abdominal binders, and post-surgical garments.
- Magnetic Closures: Magnetic closures use magnets embedded in the fabric to fasten the garment together. They offer a convenient and easy-to-use closure option, particularly for individuals with limited dexterity or strength. Magnetic closures are commonly found on compression wraps, post-surgical garments, and adaptive clothing.
- Drawstrings: Drawstrings are used to tighten or loosen the garment around the body. They provide customizable fit options and are often found on compression garments, post-surgical vests, and orthopedic braces. Drawstrings may feature cord locks or toggles to secure the closure in place.
- Buckles: Buckles provide a secure closure and are commonly used on orthopedic braces, splints, and support belts. They offer adjustable fit options and are designed to withstand movement and tension without coming undone.
- Compression Straps: Some medical garments feature compression straps with hook-and-loop closures or buckle fasteners. These straps allow for adjustable compression and secure fit, making them suitable for compression wraps, braces, and support garments.

Shoulder adjustments for the garment can include the following:
- Slide Adjusters: Slide adjusters, also known as slide buckles or sliders, consist of two small pieces of metal or plastic that slide along the length of the bra strap. By moving the adjusters up or down, the wearer can increase or decrease the length of the straps to achieve the desired fit. Slide adjusters are widely used in compression bras and offer precise adjustment options.
- Hook-and-Eye Adjusters: Similar to the closures found on bras, hook-and-eye adjusters feature hooks on one end of the strap and fabric loops (eyes) on the other end. The wearer can adjust the length of the strap by attaching the hook to different eyelets, providing multiple fit options. Hook-and-eye adjusters offer versatility and are commonly found on compression bras.
- Hook-and-Loop Adjusters: Some compression bras feature hook-and-loop adjusters on the straps, allowing for easy and quick adjustment. The hook-and-loop strips adhere to each other to secure the strap at the desired length. Hook-and-loop adjusters are convenient for individuals with limited mobility or dexterity.
- Ribbon or Tie Adjusters: Some compression bras feature straps made of soft fabric ribbons or ties that can be tied or untied to adjust the length. This allows for customizable fit options and may be suitable for individuals who prefer a more adjustable and flexible design.
- Elastic Stretch Adjusters: Elastic stretch adjusters are built into the bra straps, allowing them to stretch and conform to the wearer's body while providing support. These adjusters offer flexibility and comfort, making them suitable for compression bras intended for active wear or everyday use.
- Ladder Strap: The shoulder strap of the bra is typically constructed with a strip of fabric that contains a series of evenly spaced slots or openings running vertically along its length. These slots resemble the rungs of a ladder, hence the name "ladder adjustment." Attached to the end of the shoulder strap is a small plastic or metal device called a slider or adjuster. The slider has a protruding tab or bar that can be inserted into one of the slots on the ladder strap.

Fabrics used to make the torso encircling garment can include a stretchable material to provide compressive force against the torso. Other examples of such fabrics include flat knit fabrics, circular knit fabrics, antimicrobial fabrics, and moisture wicking fabrics.

Having the structures directly against the body without any barrier can allow the padding to be in full contact against the skin. Also, having only one unit to don and doff provides simplicity in many ways and convenience for the user.

Although specific advantages have been enumerated above, various embodiments may include some, none, or all of the enumerated advantages. Other technical advantages may become readily apparent to one of ordinary skill in the art after review of the following figures and description. It is understood that, although exemplary embodiments are illustrated in the figures and described herein, the principles of the present disclosure may be implemented using any number of techniques, whether currently known or not. Modifications, additions, or omissions may be made to the systems, apparatuses, and methods described herein without departing from the scope of the invention. The components of the systems and apparatuses may be integrated or separated. The operations of the systems and apparatuses disclosed herein may be performed by more, fewer, or other components and the methods described may include more, fewer, or other steps. Additionally, steps may be performed in any suitable order. As used in this document, "each" refers to each member of a set or each member of a subset of a set. It is intended that the claims and claim elements recited below do not invoke 35 U.S.C. §112(f) unless the words "means for" or "step for" are explicitly used in the particular claim. The above-described embodiments, while including the preferred embodiment and the best mode of the invention known to the inventor at the time of filing, are given as illustrative examples only. It will be readily appreciated that many deviations may be made from the specific embodiments disclosed in this specification without departing from the spirit and scope of the invention. Accordingly, the scope of the invention is to be determined by the claims below rather than being limited to the specifically described embodiments above.

## Claims

1. A truncal compression garment for use by a user having a torso and lymphatic fluid pathways, the truncal compression garment comprising:
(a) a body portion that has dimensions so as to be able to be wrapped at least partially around a length of the user's torso;
(b) a supporting band that is made of an elastic material that holds the body portion in place while being worn by the user; and
(c) an integrated structure disposed on an inside part of the body portion and configured to promote movement and transport of lymph in the user towards available and undamaged lymph vessels.

2. The truncal compression garment of Claim 1, wherein the torso has a front with a center and a side, where the integrated structure includes a plurality of elongated protrusions that define a corresponding plurality of channels therebetween, wherein the protrusions are disposed in a rayed arrangement, fanning out from the side to the center.

3. The truncal compression garment of Claim 1, wherein the integrated structure includes structures selected from a list consisting of: channeling, protrusions, chip bags, ridges, ribbing specifically designed patterns configured to benefit lymphedema, and combinations thereof.

4. The truncal compression garment of Claim 1, wherein the integrated structure includes materials selected from a list consisting of: foam, quilted fabric, silicone, gel, air, gel-infused foam, polymer beads, foam rubber, fiberfill, and combinations thereof.

5. The truncal compression garment of Claim 1, wherein the integrated structure portion covers both a left side of the user's torso and right side of the user's torso.

6. The truncal compression garment of Claim 1, wherein the integrated structure portion covers only one side of the body portion.

7. The truncal compression garment of Claim 1, wherein the integrated structure portion covers only one side the body portion and wherein the front closure is reversible.

8. The truncal compression garment of Claim 1, further comprising a closure that is configured to secure the body portion to the user, the closure being disposed on a front portion of the truncal compression garment.

9. The truncal compression garment of Claim 8, wherein the closure includes a mechanical connection selected from a list consisting of: a hook an loop fastener, a zipper, a plurality of snap buttons, a hook-and-eye, a plurality of magnetic closures, a drawstring, a buckle, a strap, and combinations thereof.

10. The truncal compression garment of Claim 1, further comprising at least one strap that fits over a shoulder of the user, so as to keep the garment in place and to provide compression.

11. The truncal compression garment of Claim 1, further comprising at least one adjustable strap made of a material selected from a list consisting of: slide adjusters, hook and eye adjusters, hook-and-loop adjusters, tie adjusters, elastic stretch adjusters, and ladder adjusters, and combinations thereof.

12. A method of relieving lymphedema in a user having a lymphatic system that transports lymphatic fluid, comprising the steps of:
(a) massaging an area of the user with a truncal compression garment so as to move the lymphatic fluid; and
(b) using protrusions in the truncal compression garment to direct transport of lymph in the user towards available and undamaged lymph vessels.
